# EUROPÄISCHE PATENTSCHRIFT

(11) **EP 1 709 931 B1**
(45) Veröffentlichungstag und Bekanntmachung des Hinweises auf die Patenterteilung: **16.11.2011**
(21) Anmeldenummer: 06007450.7
(22) Anmeldetag: 07.04.2006
(51) Int. Cl.: A61C 1/14, A61B 17/16, B23B 31/20, B23B 31/26

(54) **Motorelement, insbesondere dentalmedizinisches Handstück mit einer lösbaren Kupplung für einen Werkzeughalter**
Motor element, in particular dental hand piece with a releasable coupling for a tool holder
Organe moteur, en particulier pièce à main dentaire avec un accouplement détachable pour un porte-outil

(30) Priorität: 07.04.2005 DE 102005016044; 12.04.2005 DE 102005016870
(43) Veröffentlichungstag der Anmeldung: 11.10.2006
(73) Patentinhaber: Kaltenbach & Voigt GmbH, 88400 Biberach (DE)
(72) Erfinder: Düsing, Josef, 88299 Leutkirch (DE)
(74) Vertreter: Schmidt-Evers, Jürgen

(56) Entgegenhaltungen:
- CH-A- 610 755
- DE-A1- 1 552 197
- DE-A1- 3 402 585
- DE-A1- 3 603 301
- DE-A1- 4 406 855
- DE-C1- 3 928 211
- US-A- 2 898 119
- US-A- 4 007 528
- US-A- 4 202 102
- US-A- 5 904 451

## Beschreibung

Die Erfindung betrifft ein Motorelement, insbesondere ein dentalmedizinisches Handstück nach dem Oberbegriff des Anspruchs 1.

Ein Handstück dieser Art ist in der DE 44 06 855 A1 beschrieben. Bei diesembeschrieben. Bei diesem vorbekannten Handstück ist ein als Spannzange ausgebildeter Werkzeughalter in einer wenigstens vorderseitig hohlzylindrisch ausgebildeten Antriebswelle eingeschoben, wobei eine Kupplung zur axialen Verbindung des Werkzeughalters mit der Antriebswelle durch eine Schraubkupplung gebildet ist. Infolgedessen ist der Werkzeughalter durch ein Einschrauben mit der Antriebswelle verbunden und durch ein Losschrauben wieder lösbar, z.B. um ihn zu reinigen. Dieser Montage- bzw. Demontageaufwand ist verhältnismäßig groß. Außerdem ist es mechanisch schwierig den Werkzeughalter im eingeschraubten Zustand zu fixieren, um ein ungewolltes Rückdrehen zu vermeiden. Dabei ist zu berücksichtigen, dass im Funktionsbetrieb des Handstücks beim Ein- und Ausschalten der Antriebswelle Drehmomente entstehen, die eine aufgrund von Festschrauben beruhende Schraubensicherung lösen können, so dass die Funktion der Spannzange gestört und beeinträchtigt sein kann, ohne dass dieser Fehler direkt bemerkbar ist. Ein weiterer Nachteil besteht darin, dass ein Festschrauben verhältnismäßig schwierig ist, weil die Spannzange weitgehend in der Antriebeswelle versenkt angeordnet ist und es deshalb schwierig ist, ein Drehmoment zum Festschrauben am vorderen Ende der Spannzange abzusetzen.

Der Erfindung liegt deshalb die Aufgabe zugrunde, bei einem erfindungsgemäßen Handstück die Kupplung des Werkzeughalters bzw. die Arretierung und/oder Entarretierung der Kupplung zu verbessern.

Diese Aufgabe wird durch die Merkmale der unabhängigen Ansprüche gelöst. Vorteilhafte Weiterbildungen sind Gegenstand der Unteransprüche.

Bei dem erfindungsgemäßen Handstück gemäß Anspruch 1 ist das Arretierungsmittel von dem Werkzeug gebildet, derart, dass die Arretierung/Entarretierung durch eine Relativverschiebung zwischen dem Werkzeughalter und dem Werkzeug bewirkt wird, wobei die Relativverschiebung insbesondere axial gerichtet ist und die Kupplung ein radial bewegbares Kupplungselement aufweist. Da bei dieser Ausgestaltung das Arretierungsmittel durch das Werkzeug gebildet ist, wird die Kupplung beim Einführen des Werkzeugs in den Werkzeughalter selbsttätig arretiert, so dass es keiner besonderen Maßnahme oder Handhabung zum Arretieren bedarf. Zum Lösen der Kupplung ist es lediglich erforderlich, das Werkzeug z.B. aus dem Bereich der Kupplung herauszuziehen, wodurch die Arretierung aufgehoben wird und der Werkzeughalter demontiert werden kann.

Auch die erfindungsgemäße Ausgestaltung eignet sich sehr vorteilhaft dafür, die axial wirksame Kupplung in eine wenigstens vorderseitig hülsenförmig oder hohlzylindrisch ausgebildete Antriebswelle anzuordnen. Dabei kann auch hier die Kupplung einen so großen Abstand vom vorderen Ende der Antriebswelle haben, dass der Werkzeughalter im wesentlichen vollständig in der hohlen Antriebswelle eingeschoben ist. Darüber hinaus eignet sich auch die erfindungsgemäße Ausgestaltung sehr vorteilhaft dazu, den Werkzeughalter durch eine Spannzange zu bilden. Eine Spannzange weist mehrere, vorzugsweise drei, einander gegenüberliegend angeordnete Spannsegmente auf, zwischen denen ein Steckloch für ein Werkzeug bzw. dessen Schaft vorhanden ist.

Bei der erfindungsgemäßen Ausgestaltung weist das Werkzeug somit ein Sperrelement auf, das in der eingesteckten Stellung des Werkzeugs ein zwischen einer Kupplungsstellung und einer Freigabestellung bewegbares Kupplungselement der Kupplung in seiner Kupplungsstellung sperrt. Dies lässt sich dadurch realisieren, dass das bewegbare Kupplungselement sich in seiner Kupplungsstellung neben dem Steckloch befindet und zum Entkuppeln in den freien Querschnitt des Stecklochs bewegt wird. Dies kann nur dann erfolgen, wenn das Werkzeug nicht eingesteckt ist. Ist dagegen das Werkzeug eingesteckt, lässt sich das bewegbare Kupplungselement nicht in seine Freigabestellung bewegen, wodurch die erfindungsgemäße Arretierung geschaffen ist.

Die Erfindung eignet sich insbesondere für ein medizinisches Handstück, das je nach Verwendungsbereich zum einen von kleiner Konstruktion und zum anderen von stabiler Konstruktion sein kann. Für ein dentalmedizinisches Handstück ist eine kleine Konstruktion gefordert, um im beengten Mundraum eines Patienten die Behandlung ausführen zu können und darüber hinaus auch eine hinreichende Sicht auf die Behandlungsstelle zu gewährleisten. Aber auch für den allgemeinen medizinischen Bereich kann eine kleine Konstruktion gefordert sein, insbesondere dann, wenn ein solches Handstück in kleine Körperhöhlen eingesetzt werden soll. Die Erfindung eignet sich besonders gut für medizinische Handstücke stabiler Konstruktion, und zwar insbesondere solchen Handstücken, die in einem medizinischen oder dentalmedizinischen Labor zur Bearbeitung von künstlichen Körperteilen wie Prothesen, Abdrücken oder Modellen eingesetzt werden. Solche medizinische Handstücke, die eine sich im wesentlichen gerade erstreckende Form und einen Werkzeughalter aufweisen, in dem ein Werkzeug mit einem Schaft in eine im wesentlichen koaxialen Position einsteckbar ist, werden in der Fachsprache mit medizinisch technischen oder dentalmedizinischen Handstücken oder Arbeitshandstücken bezeichnet. Neben diesen medizinischen Anwendungen könnte der erfindungsgemäße Werkzeughalter allerdings generell bei Motorelementen bzw. motorgetriebenen Systemen mit einem Halter für ein rotierendes Werkzeug, beispielsweise auch bei HF-Motorspindeln eingesetzt werden.

Ein weiterer Vorteil der erfindungsgemäßen Ausgestaltung ist dann vorhanden, wenn die axial wirksame Kupplung durch eine Verrastungsvorrichtung gebildet ist, die vorzugsweise so funktioniert, dass beim axialen Einschieben des Werkzeughalters in das Steckloch das wenigstens eine bewegbare Kupplungselement gegen eine elastische Rückstellkraft radial in seine Freigabestellung bewegt wird und in der axialen Kupplungsendstellung selbsttätig in seine Kupplungsstellung einfedert. Dies kann z.B. dadurch bewirkt werden, dass am bewegbaren Kupplungselement und/oder am Gegenkupplungselement schräge oder gerundete Einführungsflächen angeordnet sind, die beim Einstecken des Werkzeughalters das vorbeschriebene Ausweichen des bewegbaren Kupplungselements bewirken.

In vergleichbarer Weise ist es auch vorteilhaft, durch vorbeschriebene schräge oder gerundete Ausführungsflächen ein selbsttätiges Ausweichen des wenigstens einen bewegbaren Kupplungselements beim Lösen der Kupplung durch ein axiales Herausziehen des Werkzeughalters zu bewirken. Die elastische Federkraft zum Bewegen des Kupplungselements in seine Kupplungsstellung und die Einführungsflächen bzw. Ausführungsflächen sind jeweils mit einem so großen Winkel bezüglich der Längsachse auszubilden, dass das wenigstens eine Kupplungselement sowohl beim axialen Einstecken als auch beim axialen Herausziehen mit einer handhabungsfreundlich aufbringbaren Axialkraft in seine Freigabestellung ausfedert und selbsttätig wieder einfedert.

Eine einfach herstellbare und sicher funktionierende Ausgestaltung für das wenigstens eine bewegbare Kupplungselement wird dann erreicht, wenn es an einem sich vom Werkzeughalter nach hinten erstreckenden Federarm angeordnet ist. Dabei können das Kupplungselement und der Federarm segmentförmig ausgebildet und durch Längsschlitze vom übrigen Körper des Werkzeughalters oder von wenigstens einem benachbarten bewegbaren Kupplungselement getrennt sein. Wenn dabei der Werkzeughalter durch eine Spannzange gebildet ist, ist es aus Raumausnutzungsgründen vorteilhaft, die vorbeschriebenen Längsschlitze zur Bildung eines oder mehrerer bewegbare Kupplungselemente bezüglich den Längsschlitzen einer üblichen Spannzange in Umfangsrichtung zu versetzen. Weitere Ausgestaltungsmerkmale der Erfindung begünstigen eine kleine Konstruktion und eine einfache Herstellung sowie Funktion.

Nachfolgend werden vorteilhafte Ausgestaltungen der Erfindung anhand eines Ausführungsbeispiels und Zeichnungen näher erläutert. Es zeigt:
- Fig. 1: ein erfindungsgemäßes medizinisches Handstück im axialen Längsschnitt;
- Fig. 2: den vorderen Endbereich des Handstücks in nur geringfügig abgewandelter vergrößerter Darstellung;
- Fig. 3: eine Spannzange des Handstücks im axialen Schnitt;
- Fig. 4: die Spannzange in der Unteransicht;
- Fig. 5: die Spannzange in der Draufsicht;
- Fig. 6: eine Antriebswellen-Baueinheit im axialen Längsschnitt;
- Fig. 7: die in Fig. 6 mit X gekennzeichnete Einzelheit in vergrößerter Schnittdarstellung;
- Fig. 8: eine Zugstangen-Baueinheit im axialen Schnitt;
- Fig. 9: den vorderen Endbereich des Handstücks in weiter abgewandelter Ausgestaltung im axialen Längsschnitt;
- Fig. 10: ein Handstück in weiter abgewandelter Ausgestaltung im axialen Längsschnitt;
- Fig. 11: den vorderen Endbereich des Handstücks gemäß Fig. 10 in vergrößerter Darstellung;
- Fig. 12: einen Werkzeughalter in Form einer Spannzange des Handstücks gemäß Fig. 10 und 11 in vergrößerter Darstellung;
- Fig. 13: die Spannzange gemäß Fig. 12 in der Unteransicht.

Als bevorzugtes Ausführungsbeispiel für die vorliegende Erfindung wird nachfolgend ein Handstück für ein medizinisches oder dentales Labor beschrieben. Wie allerdings bereits erwähnt wurde, kann die erfindungsgemäße Lösung allgemein bei Motorelementen, z.B. bei Motorspindeln eingesetzt werden.

Das in seiner Gesamtheit mit 1 bezeichnete Handstück eignet sich aufgrund seiner stabilen und hinreichend großen Konstruktion zur spanabhebenden Bearbeitung von Modellen, künstlichen Teilen wie Prothesen oder Abdrücken des menschlichen oder tierischen Körpers. In der Fachsprache wird ein solches Handstück 1 mit medizintechnisches oder dentaltechnisches Handstück oder Arbeitshandstück bezeichnet. Für eine Zahnbehandlung im Mundraum eines Patienten ist das Ausführungsbeispiel wegen seiner Konstruktionsgröße weniger gut geeignet. Jedoch eignet sich die beim Ausführungsbeispiel realisierte Erfindung prinzipiell auch für solche Handstücke, die sich für die Zahnbehandlung im Mundraum eines Patienten eignen. Gleiches gilt auch für den medizinischen Bereich.

Die Hauptteile des Handstücks 1 sind ein dem manuellen Ergreifen dienender Schaft 2, der sich wenigstens in seinem vorderen Bereich gerade erstreckt und beim Ausführungsbeispiel sich insgesamt gerade erstreckt, eine sich im Schaft 2 längs erstreckende Antriebswelle 3, die in einem vorderen und einem hinteren Drehlager 4, 5 drehbar und axial unverschiebbar im Schaft 2 gelagert ist, ein vorzugsweise elektrischer Antriebsmotor 6, der im Schaft 2 als Drehantrieb für die Antriebswelle 3 angeordnet ist und eine Spannvorrichtung 7 für ein andeutungsweise dargestelltes Werkzeug 8, das mit einem Werkzeugschaft 8a in ein Steckloch 9 der Spannvorrichtung 7 einsteckbar und spannbar sowie wieder lösbar ist.

Die Spannvorrichtung 7 weist einen Werkzeughalter 12 auf, der vorzugsweise durch eine sogenannte Spannzange 11 gebildet ist, die beim Ausführungsbeispiel in der Antriebswelle 3 in ihrer Längsrichtung verschiebbar gelagert ist und mehrere, z.B. drei, auf dem Umfang verteilt und endseitig angeordnete Spannsegmente 11a aufweist, die durch einen Spannmechanismus gegen das Werkzeug 8 bzw. den Werkzeugschaft 8a spannbar sind sowie durch einen von außen manuell zugänglichen Lösemechanismus 13 wieder lösbar sind. Für den Spann- und Lösevorgang ist die Spannzange 11 zwischen einer Spannstellung und einer Lösestellung längs verschiebbar. Beim Ausführungsbeispiel werden die Spannsegmente 11 a vorzugsweise beim Zurückbewegen der Spannzange 11 radial einwärts gegen das Werkzeug 8 bzw. dessen Schaft 8a zusammengedrückt. Hierzu dient ein Spannkonus 15 mit einem Innenkonus 15a in der Antriebswelle 3 und dazu passenden Außenkonusflächen 15b an den Spannsegmenten 11 a. In der nach vorne verschobenen Lösestellung der Spannzange 11 sind die Spannsegmente 11a ohne Spanndruck, so dass der Werkzeugschaft 8a in die Spannzange 11 eingesteckt bzw. herausgezogen werden kann. Damit bei fehlendem Werkzeug 8 die Spannsegmente 11 a nicht zu weit radial nach innen bewegt werden, sind an den vorderen Enden der Spannsegmente 11 a Flanschstücke 11b angeordnet, die die Bewegung der Spannzange 8 axial nach innen durch Anlage an der Antriebswelle 3 begrenzen.

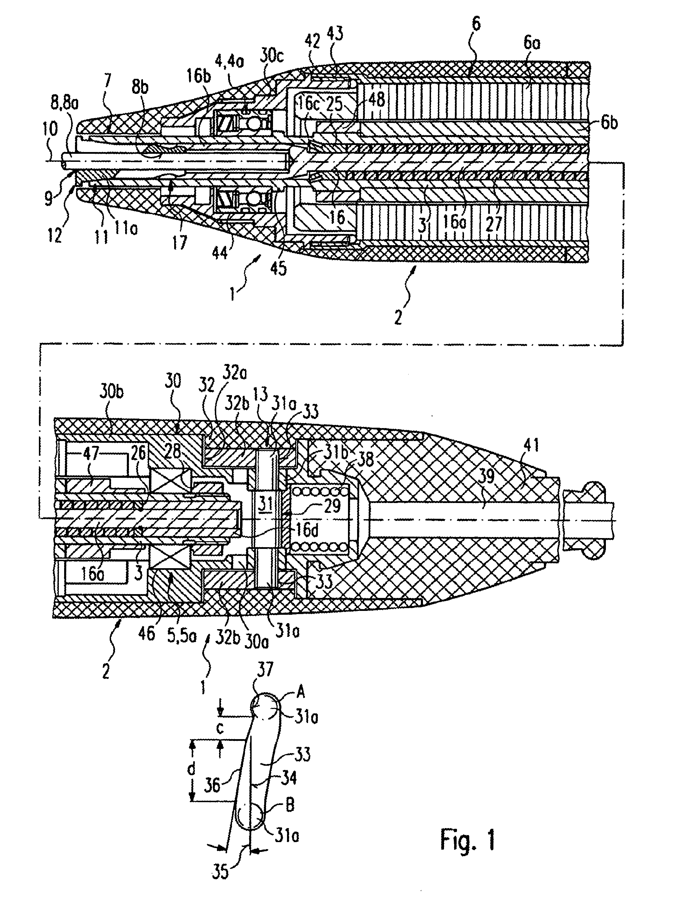

Zum längs gerichteten Hin- und Herbewegen der Spannzange 11 dient eine sich längs erstreckende Stange 16, beim Ausführungsbeispiel gemäß Fig. 1 eine Zugstange , die koaxial in der Antriebswelle 3 verschiebbar gelagert ist und an ihrem vorderen Ende lösbar mit dem hinteren Ende der Spannzange 11 verbunden ist. Hierzu ist zwischen der Stange 16 und der Spannzange 11 eine axial und formschlüssig wirksame Kupplung 17 mit einem ersten Kupplungselement 17a an dem einen zu kuppelnden Teil und einem zweiten Kupplungselement 17b an dem anderen zu kuppelnden Teil angeordnet, wobei das erste Kupplungselement 17a zwischen einer das andere Kupplungselement 17b in einer Hinterschneidung 18 formschlüssig hintergreifenden Kupplungsstellung und einer aus der Hinterschneidung 18 heraus bewegten Freigabestellung bewegbar und durch ein Arretierungsmittel 8b in der Kupplungsstellung arretierbar ist. Durch die Arretierung ist gewährleistet, dass das bewegbare Kupplungselement 17a das andere Kupplungselement 17b in der Kupplungsstellung nicht nur formschlüssig hintergreift sondern auch darin gegen ein Herausbewegen arretiert ist. Hierdurch ist die Kupplungssicherheit gewährleistet, so dass die Kupplung 17 axiale Kräfte von der Stange 16 auf die Spannzange 11 oder umgekehrt übertragen kann.

Die Erfindung ist nicht auf einen Werkzeughalter 12 in der Form einer Spannzange 11 beschränkt. Eine Spannvorrichtung 7 kann im Rahmen der Erfindung z.B. im vorderen Endbereich des Werkzeughalters 12 angeordnet sein und z.B. eine Klemmschraube aufweisen, mit der ein Werkzeug am Werkzeughalter festklemmbar ist. Deshalb wird im folgenden die Beschreibung mit dem Bauteil Werkzeughalter 12 fortgesetzt, sofern eine Spannzange 11 nicht erforderlich ist.

Im Rahmen der Erfindung kann das radial bewegbare erste Kupplungselement 17a an der Stange 16 und das radial starre zweite Kupplungselement 17b am Werkzeughalter 12 angeordnet sein. Beim Ausführungsbeispiel ist die umgekehrte Anordnung vorgesehen. Das erste Kupplungselement 17a ist am Werkzeughalter 12 und das zweite Kupplungselement 17b ist an der Stange 16 angeordnet. Ein solcher Werkzeughalter 12 weist einen hinteren Werkzeughalterabschnitt auf, mit dem er in die im vorderen Endbereich hohl ausgebildete Antriebswelle 3 bis in den Bereich der Kupplung 17 einsteckbar ist. Zur Bewegung des ersten Kupplungselements 17a in die Hinterschneidung 17c erfolgt vorzugsweise selbsttätig durch ein das erste Kupplungselement 17a in seine hintergreifende Kupplungsstellung vorspannenden Federelement. Beim Ausführungsbeispiel ist das erste Kupplungselement 17a an einem vom Werkzeughalterkörper nach hinten erstreckenden Federarm 19 angeordnet, von dem es radial nach außen absteht. Das erste Kupplungselement 17a und der Federarm 19 sind durch radiale Längsschlitze 21 vom übrigen Körper des Werkzeughalters 12 getrennt, die am zugehörigen Ende axial ausmünden.

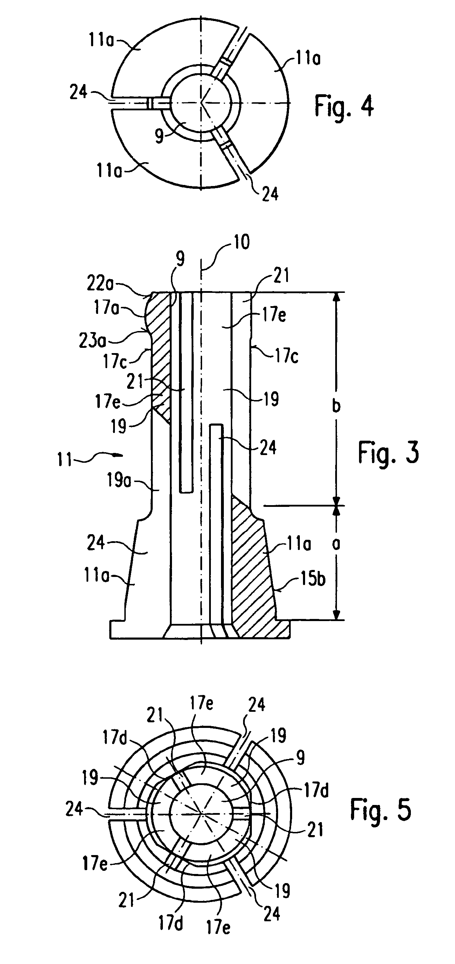

Es ist zur Erzielung einer Drehsicherung vorteilhaft, die beim Ausführungsbeispiel hinter dem zweiten Kupplungselement 17b, nämlich dem Gegenkupplungselement, vorhandene Hinterschneidung 17c mit das erste Kupplungselement 17a in beiden Umfangsrichtungen mit Bewegungsspiel begrenzenden Ausnehmungsflächen 17d (Fig. 5) auszubilden. Hierzu ergibt sich eine in beide Umfangsrichtungen formschlüssig wirksame Drehsicherung für den Werkzeughalter 12 in der Antriebswelle 3. Es ist aber auch vorteilhaft, die Hinterschneidung 17c als Ringnut bzw. als sich in den Spannsegmenten 11a in der Umfangsrichtung fortsetzende Nut auszubilden. In einem solchen Fall ist zwar keine Drehsicherung im Bereich der Kupplung 17 vorhanden, jedoch wird hierdurch der Vorteil erreicht, dass der Werkzeughalter 12 in jeder wahlweisen Drehstellung in die Kupplung 17 eingeschoben und gekuppelt werden kann. Eine Drehsicherung kann dabei auf eine andere Art und Weise realisiert sein. Im Falle der Ausbildung des Werkzeughalters 12 als Spannzange 11 ist eine Drehsicherung durch den Klemmkonus der Spannzange 11 geschaffen.

Um die Montage und/oder Demontage des Werkzeughalters 12 zu erleichtern, ist es vorteilhaft, dass erste Kupplungselement 17a und/oder das zweite Kupplungselement 17b mit vorderen bzw. hinteren schrägen oder gerundeten Einführungsflächen 22a, 22b bzw. Ausführungsflächen 23a, 23b auszubilden, die bezüglich der Längsmittelachse und Drehachse 10 der Antriebswelle 3 eine so große Neigung aufweisen, dass der Werkzeughalter 12 mit einer handhabungsfreundlich aufbringbaren Axialkraft in die Kupplung 17 eingeschoben und/oder aus der Kupplung 17 herausgezogen werden kann, wobei das bewegbare Kupplungselement 17a selbsttätig ausfedert. Hierdurch ist eine Verrastungsvorrichtung gebildet, deren Verrastungskräfte so groß sind, dass im Funktionsbetrieb die axiale Kupplung des Werkzeughalters 12 gewährleistet ist und für eine Montage und/oder Demontage des Werkzeughalters 12 die Kupplung 17 mit einem axialen manuell aufbringbaren Kraftaufwand zu überdrücken ist.

Beim Ausführungsbeispiel, bei dem mehrere, vorzugsweise drei, bewegbare erste Kupplungselemente 17a mit zugehörigen zweiten Kupplungselementen 17b mit Hinterschneidungen 17c auf dem Umfang verteilt angeordnet sind, sind die bewegbaren Kupplungselemente 17a mit den zugehörigen Federarmen 19 jeweils als einander gleiche endseitig angeordnete Segmente ausgebildet, die durch vorzugsweise drei endseitig ausmündende Längsschlitze 21 voneinander getrennt sind. Die in die Umfangsrichtung gerichtete Breite der Längsschlitze 21 ist so groß, dass die bewegbaren Kupplungselemente 17a jeweils ausfedern können, wobei die vorhandene Breite der Längsschlitze 21 sich jeweils etwas verringert.

Wenn der Werkzeughalter 12 als eine Spannzange 11 mit mehreren, vorzugsweise drei, auf dem Umfang verteilt angeordneten Spannsegmenten 11a ausgebildet ist, die durch Längsschlitze 24 voneinander getrennt und an dadurch gebildeten Federarmen 19a angeordnet sind, ist es zur Verringerung der Baulänge vorteilhaft, die Längsschlitze 21 bezüglich den Längsschlitzen 24 in die Umfangsrichtung versetzt zueinander anzuordnen, wobei die Längsschlitze 21, 24 einander überlappen können, wie es Fig. 3 deutlich zeigt.

Der hülsenförmige Werkzeughalter 12 weist ein hohlzylindrisches Steckloch 9 auf, einen vorderen konischen Längsabschnitt a und einen sich von diesem nach hinten erstreckenden im wesentlichen hohlzylindrischen Längsabschnitt b, die jeweils durch die vorbeschriebenen Segmente gebildet sind. Die Querschnittsform und -größe des Stecklochs 9 ist mit geringem Bewegungsspiel an die Querschnittsform und -größe des Werkzeugs 8 bzw. Werkzeugschaftes 8a angepasst. Die Wandabschnitte des vorzugsweise hohlzylindrischen Stecklochs 9 bilden somit im Bereich der Spannsegmente 11a hohlzylinderabschnittförmige Spannflächen und im Bereich der Kupplungssegmente hohlzylinderabschnittförmige Anschlagflächen, mit denen die Kupplungssegmente 7e an der Umfangsfläche des Werkzeugs 8 bzw. Schaftes 8a anliegen.

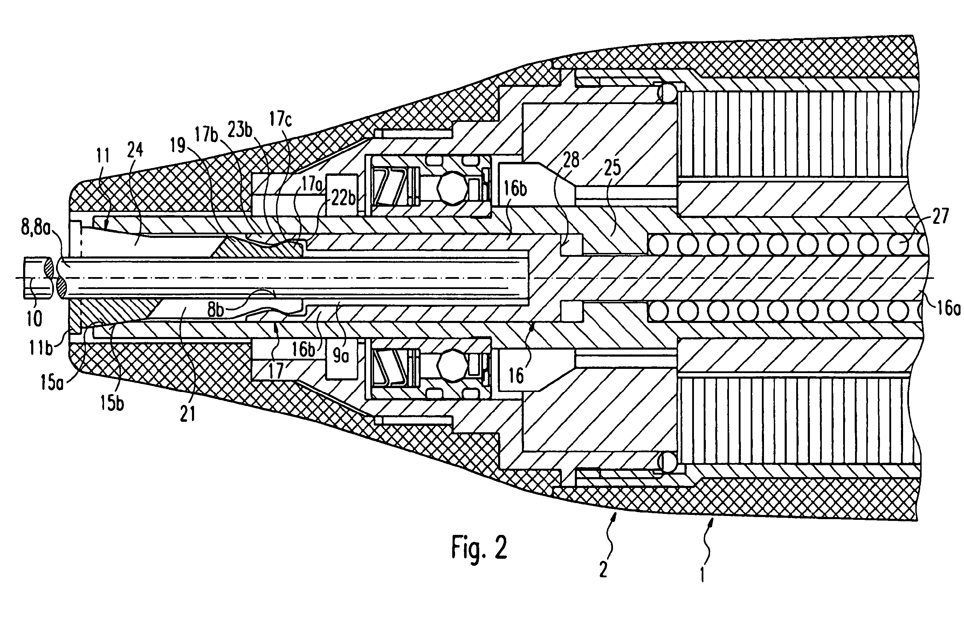

Die Ausgestaltungen gemäß Fig. 1 bzw. 2 zum einen und Fig. 5 zum anderen unterscheiden sich durch die Mantelflächenform der Kupplungselemente 17e. Gemäß Fig. 1 bzw. 2 sind die Kupplungselemente 17e vor der zugehörigen Hinterschneidung 17c verdickt, wobei die Hinterschneidungen 17c im Längsquerschnitt konkav gerundet ist. Gemäß Fig. 5 erstreckt sich die Hinterschneidung 17c vor den konvexen Kupplungselementen 17a im wesentlichen zylindrisch nach vorne.

Die Stange 16 erstreckt sich beim Ausführungsbeispiel von der Kupplung 17 nach hinten längs durch die hülsenförmige Antriebswelle 3 hindurch, wobei sie vorzugsweise das hintere Ende der Antriebswelle 3 überragt. Hinter dem vorderen Endbereich der Stange 16, in dem ein Steckloch 9a angeordnet ist, erstreckt sich die Stange 16 mit einem im Querschnitt verjüngten Stangenabschnitt 16a, der in seinem vorderen Endbereich in einer Führungsbuchse 25 in der Antriebswelle 3 längs verschiebbar geführt ist. Zwischen dem hinteren Ende der Führungsbuchse 25 und einer nach vorne gerichteten Schulterfläche 26 im hinteren Endbereich der Stange 16 befindet sich eine Druckfeder 27 in Form einer Wendelfeder auf dem verjüngten Stangenabschnitt 16a, die die Stange 16 nach hinten vorspannt. Bei demontiertem Werkzeughalter 11 liegt der verdickte vordere Endbereich 16b der Stange 16 mit einer rückseitigen Schulterfläche 16c an der Führungsbuchse 25 an. Die davon rückseitig angeordnete Schulterfläche 26 kann durch eine auf den Stangenabschnitt 16a aufgeschobene und darauf fixierte Buchse 16d gebildet sein, mit der der hintere Endbereich der Stange 16 in der hülsenförmigen Antriebswelle 3 axial verschiebbar gelagert ist. Die Führungsbuchse 25 in Fig. 2 weicht von den übrigen Ausführungsbeispielen insofern ab, als sie durch einen inneren Ringansatz der Antriebswelle 3 gebildet ist.

In der Normalstellung ist die durch die Feder 27 nach hinten vorgespannte Stange 16 in ihrer nach hinten gerichteten Bewegung dadurch begrenzt, dass bei einem in die Spannzange 11 eingesteckten Werkzeug 8 der Außenkonus der Spannzange 11 am Innenkonus der Antriebswelle 3 anliegt. Zum Lösen des Werkzeugs 8 wird die Stange 16 durch den Betätigungsmechanismus 13 nach vorne bewegt. Dies kann durch ein Druckglied 29 erfolgen, das z. B. als vorzugsweise zylindrischer Querstift 31 ausgebildet ist, der durch den Betätigungsmechanismus 13 nach vorne gegen das hintere Ende der Stange 16 (Teil 16a und/oder 16d) bewegbar ist. Beim Ausführungsbeispiel ist der Querstift 31 im Bereich seiner z. B. verjüngten Enden 31 a in Nuten 33 verschiebbar gelagert, die sich mit einer Steigung in der Umfangsrichtung des Handstücks 1 in einer Betätigungshülse 32 befinden, die in einer Ringnut 32a des Handstücks 1 axial unverschiebbar und in Umfangsrichtung drehbar gelagert ist. Eine der beiden Nuten 33 ist in Fig. 1 als Abwicklung beispielhaft dargestellt. Die vorzugsweise in einer Innenbuchse 32b, insbesondere aus verschleißfestem Material, z.B. Stahl, angeordneten Nuten 33 erstrecken sich in der Umfangsrichtung derart schräg oder gerundet, dass ausgehend von der in Fig. 1 dargestellten Spannstellung bei einer Relativverdrehung der Betätigungshülse 32 das Druckglied 29 nach vorne verdrängt wird und dabei die Stange 16 nach vorne bewegt und die Spannzange 11 löst. In dieser Lösestellung befinden sich die Enden 31a des Druckglieds 29 in den Endbereichen B der Nuten 33. Dabei kann die Anordnung so getroffen sein, dass der der Lösestellung zugehörige Endbereich B der Nuten 33 nicht schräg sondern in die Umfangsrichtung gerichtet ist, so dass ein selbsttätiges Rückdrehen der Betätigungshülse 32 aufgrund der Federspannung und ggf. aufgrund der Wirkung von Vibrationen, verhindert ist. Die bezüglich einer zugehörigen, rechtwinklig zur Längsmittelachse gerichteten Querebene 35 schräg nach vorne verlaufende Neigung 36 kann im Bereich der Länge der Nuten 33 unterschiedlich sein. Beim Ausführungsbeispiel ist ausgehend vom der Spannstellung zugehörigen Endbereich A ein verhältnismäßig stark geneigter Neigungsverlauf c vorgesehen, an den sich ein weniger stark geneigter Neigungsverlauf d anschließt, an den sich ein in die Umfangsrichtung gerichteter Endbereich B der Nuten 33 anschließt. In den Endbereichen B der Nuten 33, in denen sich die Druckgliedenden 31a in einer der Spannstellung entsprechenden Stellung befinden, sind vorzugsweise Rastvertiefungen 37 vorgesehen, in denen die Enden 31a einrasten können, und die beim Ausführungsbeispiel nach vorne gerichtet sind. Durch eine hinter dem Druckglied 29 angeordnete Feder 38, bspw. eine Wendel-Druckfeder, lässt sich das Druckglied 29 nach vorne vorspannen und somit auch in die Rastvertiefungen 37 vorspannen. Beim Drehen der Betätigungshülse 32 in die Lösestellung B werden das Druckglied 29, die Stange 16 und die Spannzange 11 in die Lösestellung vorbewegt. Beim Zurückdrehen der Betätigungshülse 32 in die Spannstellung A wird das Druckglied 29 wieder in die beabstandete Spannstellung (Freistellung) zurückbewegt, in der es die Stange 16 freigibt und die Spannzange 11 aufgrund der vorhandenen axialen Federkraft gespannt wird.

Bei den vorbeschriebenen Vor- und Rückbewegungen des Druckglieds 29 wird es z. B. mittels auf ihm sitzenden Gleithülsen 31b in Längsnuten 30a einer tragenden Umfangswand 30b eines Handstück-Gehäuses 30 längs geführt.

Als Drehantrieb für die Antriebswelle 3 ist der Elektromotor 6 vorgesehen, der im Handstück 1 angeordnet ist und z.B. mit einem hülsenförmigen Stator 6a innen an der Umfangswand des vorhandenen Handstückgehäuses angeordnet ist und mit seinem hülsenförmigen Rotor 6b auf der Antriebswelle 3 sitzt. Eine andeutungsweise dargestellte elektrische Stromleitung 39 erstreckt sich von hinten durch eine flexible Kabeldurchführung 41 aus elastisch verformbarem Material, die mit einem hinteren Gehäuseabschnitt des Handstückgehäuses verbunden ist.

Das Gehäuse 30 besteht aus zwei tragenden Umfangswänden, nämlich der hinteren längeren Umfangswand 30b und einer vorderen kürzeren Umfangswand 30c, die im vorderen Bereich des Gehäuses 30 einander überlappen und in diesem Bereich durch ein Außengewinde und ein darin einfassendes Innengewinde miteinander verschraubt sind und an einer Teilungsfuge 42 aneinanderliegen. Die z. B. hinter der Teilungsfuge 42 angeordnete Verschraubung ist mit 43 bezeichnet. Die hintere Umfangswand 30b ist im wesentlichen hohlzylindrisch ausgebildet und in ihrem Bereich ist der Stator 6a angeordnet. Der vordere Handstückabschnitt und die vordere Umfangswand 30c verjüngen sich zum vorderen Ende des Handstücks 1 hin.

Das vordere Drehlager 4 ist in der vorderen Umfangswand 30c angeordnet und durch ein Wälzlager 4a gebildet, das außen in einer nach hinten offenen Lagerbohrung 44 in der vorderen Umfangswand 30c sitzt und innen auf der hohlen Antriebswelle 3 sitzt, wobei es rückseitig von einem Ringbund 45 auf der Antriebswelle 3 begrenzt ist.

Das hintere Drehlager 5 ist ebenfalls durch ein Wälzlager gebildet, das außen in einer nach vorne offenen Lagerbohrung 46 im hinteren Endbereich der hinteren Umfangswand 30b sitzt und innen auf der Antriebswelle 3 sitzt. Der Innenring des hinteren Wälzlagers 5a ist an seiner Vorderseite durch den Rotor 6b oder eine darin anliegende Zwischenhülse 47 begrenzt. Der Rotor 6b ist an seinem vorderen Ende durch einen Ringbund 48 auf der Antriebswelle 3 begrenzt, der auch von dem Ringbund 45 gebildet sein könnte. Rückseitig vom hinteren Wälzlager 5a befindet sich eine Gewindemutter 49, die von hinten auf einen Gewindeabschnitt der Antriebswelle 3 aufgeschraubt ist und den Innenring sowie die weiteren vorderseitig von diesen auf der Antriebswelle 3 angeordneten Ringteilen gegen die Ringbund 48 drückt und axial festliegt.

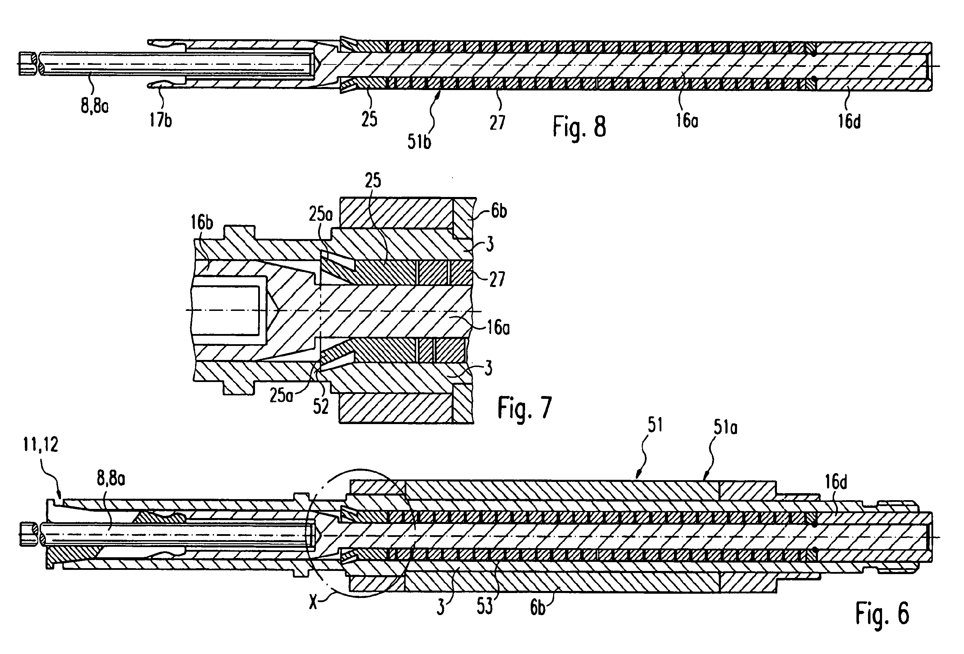

Dabei bilden die Antriebswelle 3 mit dem Rotor 6b und der Stange 16 mit der Druckfeder 27 eine vormontierbare Baueinheit 51, siehe Fig. 6.

Beim vorliegenden Ausführungsbeispiel besteht diese Baueinheit 51 aus zwei Baueinheiten 51 a, 51b, wobei die Baueinheit 51 a die Antriebswelle 3, den Rotor 6b, umfaßt, während die zweite Baueinheit 51b die Stange 16, die Buchse 16d, die Druckfeder 27 und die Führungsbuchse 25 umfaßt.

Zur axialen Positionierung dieser Baueinheit 51b ist in der Antriebswelle 3 hinter dem verdickten Endbereich 16b eine oder mehrere nach hinten gerichtete Schulterflächen 52 vorgesehen, gegen die die Führungsbuchse 25 in Richtung nach vorne begrenzt und positioniert ist. Die Druckfeder 27 ist rückseitig an der Buchse 16d abgestützt, und sie spannt die Führungsbuchse 25 nach vorne vor, wobei sie aufgrund der axialen Abstützung der Führungsbuchse 25 an der wenigstens einen Schulterfläche 52 die Stange 16 nach hinten vorspannt. Im montierten Zustand ist dabei die Stange 16 oder die Buchse 16d rückseitig am Druckglied 29 abgestützt. Diese Abstützung kann aber auch dadurch erfolgen, daß die Spannzange die Spannsegmente 11a oder die Flanschstücke 11b rückseitig durch die Antriebswelle 3 begrenzt sind.

Beim vorliegenden Ausführungsbeispiel ist die innere Baueinheit 51b von vorne in die hohle Antriebswelle 3 einsteckbar und montierbar. Dies wird dadurch erreicht, daß die Führungsbuchse 25 an ihrem vorderen Ende einen oder mehrere auf dem Umfang verteilt angeordnete Federarme 25a aufweist, deren vordere Enden im entspannten Zustand bezüglich der Längsmittelachse 10 ein Radialmaß einnehmen, das größer ist, als das Radialmaß des vorzugsweise auf seiner gesamten Länge zylindrischen Aufnahmelochs 53 in der Antriebswelle. Die Schulterfläche 52 ist durch eine Ringnut im Aufnahmeloch 53 gebildet.

Zur Montage der inneren Baueinheit 51b in die Antriebswelle 3 wird die Baueinheit 51b von vorne in das Aufnahmeloch 53 eingeschoben, bis der oder die dabei einfedernden Federarme 25a hinter der wenigstens einen Schulterfläche 52 ausfedern und die Baueinheit 51a nach vorne an der wenigstens einen Schulterfläche 52 positionieren.

Im demontierten Zustand liegt aufgrund der Federspannung der Druckfeder 27 die Führungsbuchse 25 an der ihr vorgeordneten Schulterfläche 16c an. Im montierten Zustand ist dagegen zwischen der Schulterfläche 16c und der Führungsbuchse 25 ein axiales Bewegungsspiel auch im gespannten, d. h. nach hinten verschobenen Zustand der Spannsegmente 11a vorhanden, damit die radial einwärts gerichtete Spannwirkung der Spannsegmente 11 a nicht beeinträchtigt wird.

Für eine Demontage der Baueinheit 51b wird nach einer Entfernung des Werkzeughalters 9 bzw. der Spannsegmente 11a die Stange 16 nach hinten herausgeschoben, wobei die Federarme 25a unbeschädigt einfedern.

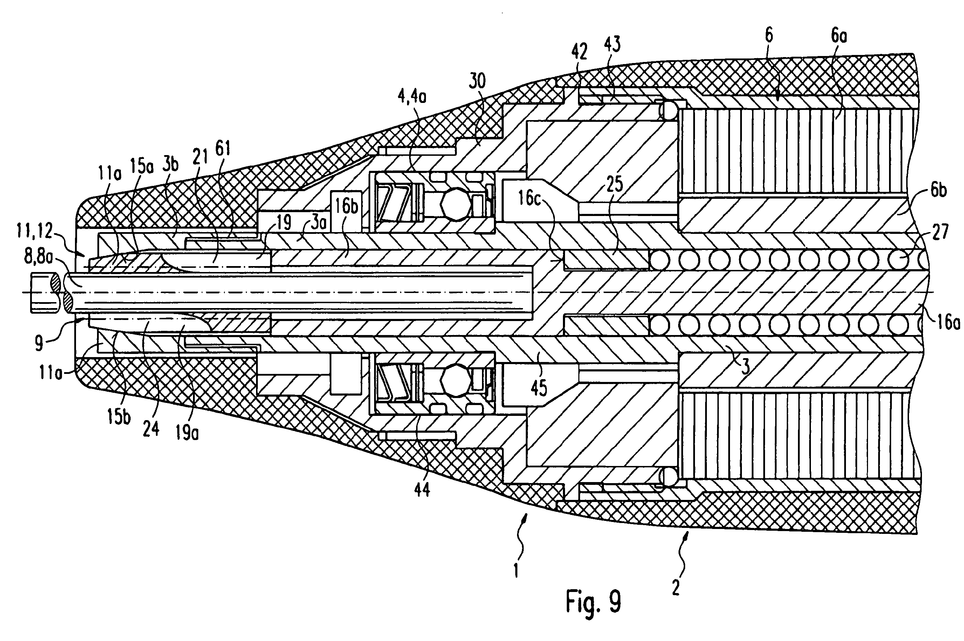

Beim Ausführungsbeispiel nach Fig. 9, bei dem gleiche oder vergleichbare Teile mit gleichen Bezugszeichen versehen sind, weist das Handstück 3 einen Werkzeughalter 12 auf, der durch eine Druck-Spannzange 11 gebildet ist, die dadurch betätigt wird, dass die Stange 16 für einen Spannvorgang nicht zurückgezogen sondern vorgeschoben wird und einen nach vorne gerichteten Druck auf die Spannzange ausübt. Auch bei dieser Ausgestaltung sind eine Gruppe, z. B. durch Spannsegmente 11a gebildete, Spannelemente mit zusammenwirkenden Konusflächen um das Steckloch 9 verteilt im Werkzeughalter 12 angeordnet und für einen Spannvorgang gegen das Steckloch 9 bewegbar, jedoch sind die Konusflächen, hier der Innenkonus 15a in der Antriebswelle 3 und die Außenkonusflächen 15b an den Spannsegmenten 11a zueinander passend nach vorne konvergent ausgebildet. Diese Konusteile sind vorzugsweise im vorderen Bereich der Spannzange 11 angeordnet.

Bei dieser Ausgestaltung ist eine Kupplung zwischen der Spannzange 11 und der Stange 16 nicht erforderlich, da eine Zugwirkung beim Spannen nicht stattfindet. Der Druck, der z.B. stumpf von hinten gegen die Spannzange 11 drückenden Stange 16, bei der es keiner Kupplung bedarf, reicht für die Spannfunktion aus. Die Spannzange 11 ist zwischen dem Innenkonus 15a und der Stange 16 auch formschlüssig unverlierbar gehalten. Auch bei dieser Ausgestaltung sind die Spannsegmente 11a an Federarmen 19a angeordnet, die durch auf dem Umfang verteilt angeordnete und nach vorne ausmündende Längsschlitze 24 gebildet sind. Dabei können auch hier nach hinten ausmündende Längsschlitze 21 angeordnet sein, die den Spannzangenkörper hinten in Segmente unterteilen.

Um die Montage der Spannzange 11 zu erleichtern, ist die in diesem Bereich hülsenförmig ausgebildete Antriebswelle 3 hinter dem Innenkonus 15a quer geteilt, und die Antriebswellenteile 3a, 3b sind durch eine lösbare Verbindung 61, insbesondere eine Schraubverbindung, miteinander verbunden.

Bei dieser abgewandelten Ausgestaltung ist die Stange 16 durch die Druckfeder 27 nach vorne in ihre Spannstellung vorgespannt. Die Spannzange 11 wird durch eine Zugbewegung der Stange 16 nach hinten gelöst, die durch den Lösemechanismus 13 erzeugbar ist, der sie entgegen der Kraft der Druckfeder 27 nach hinten bewegt.

Hierzu können z. B. die Nuten 33 eine entgegengesetzte Steigung haben, so dass das Druckglied 29 zum Lösen nach hinten bewegt wird und die Stange 16 nach hinten zieht, z. B. dadurch, dass die Stange 16 das Druckglied 29 hintergreift und dabei in einem Loch durchgreift und mit einem Kopfteil 16e hintergreift, so dass die Feder 27 die Spannelemente durch die Stange 16 nach vorne schiebt und spannt. Insoweit ist die Druck-Spannzange 11 in umgekehrter Weise wirksam wie die Zug-Spannzange 11 gemäß Fig. 1

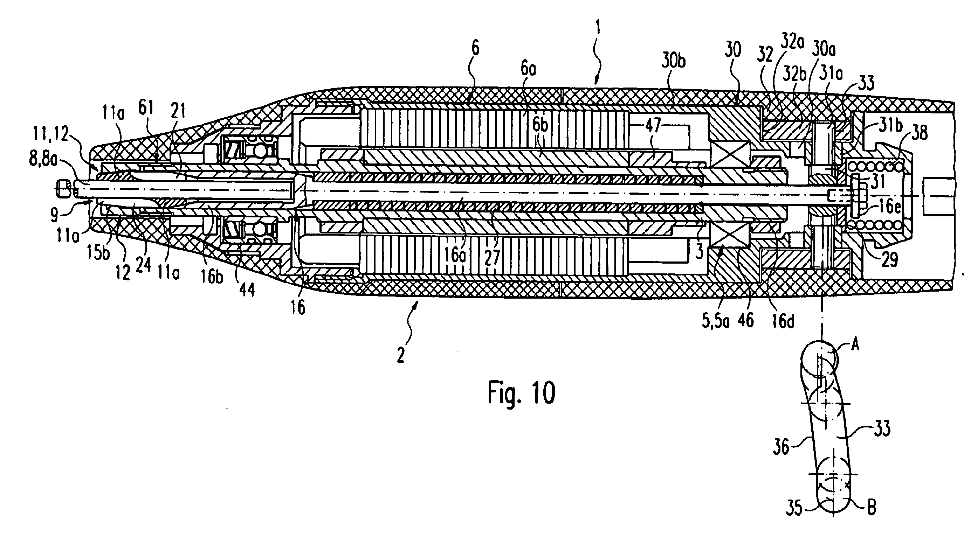

Die Spannwirkung dieses Werkzeughalters 12 lässt sich dadurch verbessern, gemäß dem Ausführungsbeispiel nach Fig. 10 dass zwei Gruppen von Spannelementen vorgesehen sind, die axial hintereinander und jeweils um das Steckloch 9 herum angeordnet und gegen das Steckloch 9 bewegbar sind. Dies lässt sich durch eine gemeinsame Druckbeaufschlagung der Gruppen erreichen, und zwar insbesondere dann in einfacher Weise, wenn in jeder Gruppe Spannsegmente angeordnet sind, von denen die Spannsegmente 11a der vorderen Gruppe nach vorne konvergente Außenkonusflächen 15b und die der hinteren Gruppe nach hinten konvergente Außenkonusflächen 15b aufweisen, wobei die Stange 16 an ihrem vorderen Ende ein insbesondere durch einen Innenkonus 15c gebildetes gemeinsames Druckglied aufweist, das mit den hinteren Außenkonusflächen 15b zusammenwirkt. Hierbei wirkt der Druck der Stange 16 gleichzeitig auf alle Spannelemente.

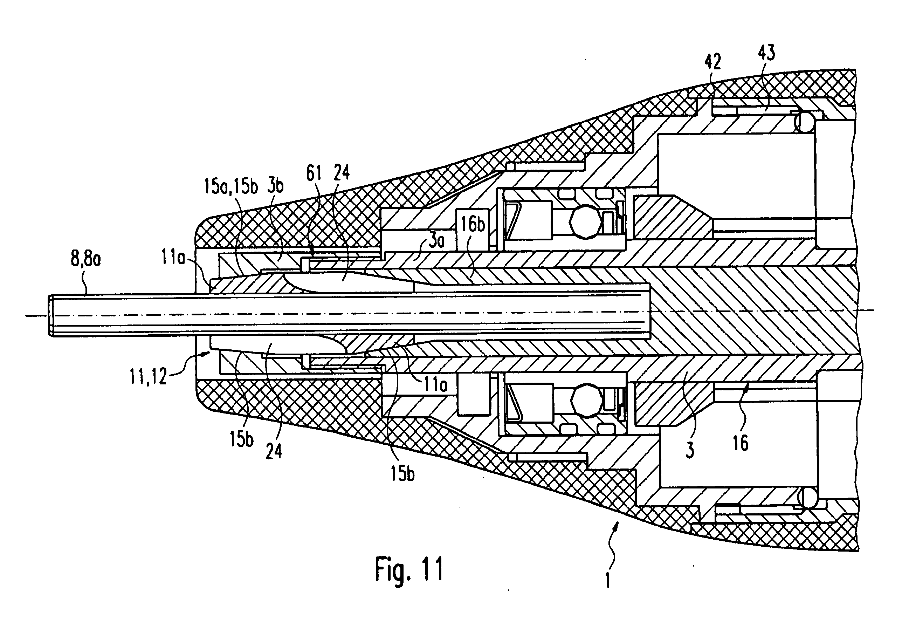

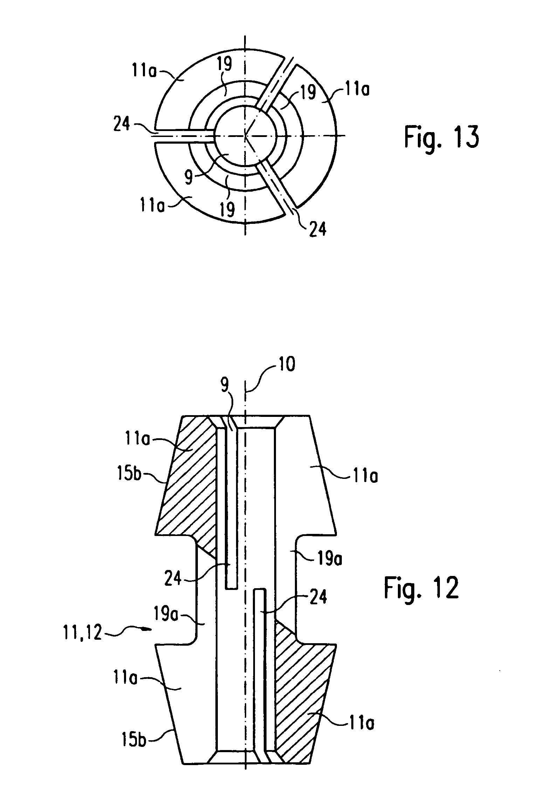

Wie insbesondere Fig. 11 und 12 zeigen, sind bei diesem Werkzeughalter 12 an beiden axialen Enden jeweils eine Gruppe von z. B. drei Spannsegmenten 11a jeweils an einem Federarm 19a gehalten und somit radial einwärts gegen die Federarmkraft bewegbar. Die Spannsegmente 11a und die Federarme 19a sind durch Längsschlitze 24 im Hülsenkörper gebildet, die jeweils endseitig ausmünden und deshalb den radialen Federweg gewährleisten. Die Längsschlitze 24 und die Spannsegmente 11a sind vorzugsweise in der Umfangsrichtung versetzt zueinander angeordnet. Hierdurch ist es möglich, die Schlitze 24 einander axial überlappend anzuordnen, insbesondere im mittleren Bereich des Hülsenkörpers und somit im Bereich zwischen den Gruppen der Spannsegmente 11a bzw. Spannelemente.

Der Werkzeughalter ist - wie bereits erwähnt - nicht auf den Einsatz in medizinischen, zahnmedizinischen oder zahntechnischen Handstücken beschränkt. Stattdessen bringt die vorliegende Erfindung grundsätzlich gesehen immer dann Vorteile mit sich, wenn ein Werkzeughalter für ein rotierendes Werkzeug lösbar mit einer - insbesondere von einem Elektromotor angetriebenen - Antriebswelle gekoppelt werden soll. Dementsprechend wäre neben einem medizinischen Einsatz auch die Verwendung des zuvor beschriebenen Werkzeughalters in anderen Systemen, beispielsweise in HF-Motorspindeln und dgl. denkbar.

## Patentansprüche

1. Motorelement (1), insbesondere dentaltechnisches oder dentalmedizinisches Handstück oder Motorspindel, das wenigstens in seinem vorderen Bereich einen länglichen Schaft (2) aufweist, in dem eine Antriebswelle (3) drehbar gelagert ist, mit einem Werkzeughalter (12) für ein Werkzeug (8), mit einer lösbaren Kupplung (17) zwischen der Antriebswelle (3) und dem Werkzeughalter (12), und mit einem Arretierungsmittel (8b) für die Kupplung (17), um deren Lösbarkeit zu unterbinden, wobei die Antriebswelle (3) wenigstens in ihrem vorderen Endbereich die Form einer Hülse aufweist und der Werkzeughalter (12) wenigstens mit einem hinteren Werkzeughalterabschnitt in die Hülse einsteckbar ist, und
wobei die Kupplung (17) in der Hülse angeordnet ist und der Werkzeughalter (12) ein Steckloch (9) aufweist, in das das Werkzeug (8) bis in den Bereich der Kupplung (17) einsteckbar ist,
**dadurch gekennzeichnet,**
**dass** die Kupplung (17) ein radial bewegbares erstes Kupplungselement (17a) aufweist, das zwischen einer neben dem Steckloch (9) angeordneten und ein zweites, radial unbewegliches, Kupplungselement (17b) axial hintergreifenden Kupplungsstellung und einer Freigabestellung bewegbar ist, in der es in das Steckloch hineinragt.

2. Motorelement nach Anspruch 1,
**dadurch gekennzeichnet,**
**dass** das erste Kupplungselement (17a) durch die Kraft einer Feder in seine Kupplungsstellung vorgespannt ist.

3. Motorelement nach Anspruch 2,
**dadurch gekennzeichnet,**
**dass** das erste Kupplungselement (17a) an einem sich im Wesentlichen axial erstreckenden Federarm (19) angeordnet ist.

4. Motorelement nach einem der vorhergehenden Ansprüche,
**dadurch gekennzeichnet,**
**dass** das erste Kupplungselement (17a) am Werkzeughalter (12) angeordnet ist, vorzugsweise in dessen hinteren Bereich.

5. Motorelement nach einem der vorhergehenden Ansprüche,
**dadurch gekennzeichnet,**
**dass** an den vorderen und/oder hinteren Enden des ersten Kupplungselements (17a) und/oder zweiten Kupplungselements (17b) schräge oder gerundete Einführungsflächen (22a, 22b) bzw. Ausführungsflächen (23a, 23b) angeordnet sind, die ein selbsttätiges Ausweichen des bewegbaren Kupplungselements (17a) beim Einstecken oder Herausziehen des Werkzeugs aus dem Bereich der Kupplung (17) bewirken.

6. Motorelement nach Anspruch 5,
**dadurch gekennzeichnet,**
**dass** die Einführungsflächen (23a, 23b) und/oder Ausführungsflächen (23a, 23b) S-förmig geformt sind.

7. Motorelement nach einem der vorhergehenden Ansprüche,
**dadurch gekennzeichnet,**
**dass** der Werkzeughalter (12) durch eine Spannzange (11) mit mehreren auf dem Umfang verteilt angeordneten und durch endseitig ausmündende Längsschlitze (24) getrennte Spannsegmente (11a) im zugehörigen Endbereich der Spannzange (11) gebildet ist, wobei die Spannzange (11) in der Umfangsrichtung versetzte und am anderen Ende der Spannzange (11) ausrnündende zweite Längsschlitz (21) aufweist, die dazwischen angeordnete Segmente der Spannzange (11) begrenzen, wobei die ersten und die zweiten Längsschlitze (24, 21) im Bereich ihrer einander zugewandten Enden einander überlappen.

8. Motorelement nach Anspruch 7,
**dadurch gekennzeichnet,**
**dass** ein oder mehrere der Segmente jeweils ein erstes Kupplungselement (17a) bilden.

## Claims

1. A motor element (1), in particular a dental technical or dental medical hand piece or motor spindle, which at least in its front region has an elongated shank (2) in which a drive shaft (3) is rotatably mounted, having a tool-holder (12) for a tool (8), having a disconnectable coupling (17) between the drive shaft (3) and the tool-holder (12), and having a locking means (8b) for the coupling (17) in order to prevent the latter from being disconnectable,
wherein the drive shaft (3) at least in its front end region has the form of a sleeve, and the tool-holder (12) can be plugged into the sleeve at least with a rear tool-holder section, and
wherein the coupling (17) is arranged in the sleeve, and the tool-holder (12) has a plug hole (9), into which the tool (8) can be plugged as far as the region of the coupling (17),
**characterised in that**
the coupling (17) has a radially movable first coupling element (17a) which can be moved between a coupling position, where it is arranged next to the plug hole (9) and axially engages behind a second radially immovable coupling element (17b), and a position of release in which it projects into the plug hole.

2. A motor element according to claim 1,
**characterised in that**
the first coupling element (17a) is pretensioned into its coupling position by the force of a spring.

3. A motor element according to claim 2,
**characterised in that**
the first coupling element (17a) is arranged on a spring arm (19) that extends substantially axially.

4. A motor element according to one of the preceding claims,
**characterised in that**
the first coupling element (17a) is arranged on the tool-holder (12), preferably in its rear region.

5. A motor element according to one of the preceding claims,
**characterised in that**
arranged at the front and/or rear ends of the first coupling element (17a) and/or second coupling element (17b) there are oblique or rounded lead-in faces (22a, 22b) or lead-out faces (23a, 23b) respectively that give rise to automatic yielding of the movable coupling element (17a) when the tool is plugged in or drawn out from the region of the coupling (17).

6. A motor element according to claim 5,
**characterised in that**
the lead-in faces (23a, 23b) and/or lead-out faces (23a, 23b) are formed so that they are S-shaped.

7. A motor element according to one of the preceding claims,
**characterised in that**
the tool-holder (12) is formed by a collet chuck (11) having a plurality of clamping segments (11a) that are arranged so that they are distributed over the periphery and are separated by longitudinal slots (24), which open out on the end side, in the associated end region of the collet chuck (11), with the collet chuck (11) having second longitudinal slots (21) that are staggered in the circumferential direction and open out at the other end of the collet chuck (11) and delimit segments of the collet chuck (11) arranged in between, with the first and the second longitudinal slots (24, 21) overlapping each other in the region of their facing ends.

8. A motor element according to claim 7,
**characterised in that**
one or a plurality of the segments in each case forms a first coupling element (17a).

## Revendications

1. Organe moteur (1), en particulier pièce à main de technique ou de médecine dentaire ou broche motorisée qui présente au moins dans sa zone avant une tige (2) oblongue, dans laquelle est logé de manière rotative un arbre d'entraînement (3), avec un porte-outil (12) pour un outil (8), un accouplement amovible (17) entre l'arbre d'entraînement (3) et le porte-outil (12), et un moyen d'arrêt (8b) pour l'accouplement (17) afin de supprimer sa capacité de détachement, sachant que l'arbre d'entraînement (3) présente la forme d'une douille au moins dans sa zone d'extrémité avant et le porte-outil (12) peut être inséré au moins par une section de porte-outil arrière dans la douille, et
sachant que l'accouplement (17) est disposé dans la douille et le porte-outil (12) présente un trou d'insertion (9), dans lequel l'outil (8) peut être inséré jusque dans la zone de l'accouplement (17),
**caractérisé en ce que** l'accouplement (17) présente un premier élément d'accouplement (17a) mobile radialement qui peut être déplacé entre une position d'accouplement disposée à côté du trou d'insertion (9) et venant en prise axialement derrière un second élément d'accouplement (17b) immobile dans le sens radial et une position de libération, dans laquelle il pénètre dans le trou d'insertion.

2. Organe moteur selon la revendication 1, **caractérisé en ce que** le premier élément d'accouplement (17a) est précontraint par la force d'un ressort dans sa position d'accouplement.

3. Organe moteur selon la revendication 2, **caractérisé en ce que** le premier élément d'accouplement (17a) est disposé sur un bras à ressort (19) s'étendant sensiblement dans le sens axial.

4. Organe moteur selon l'une quelconque des revendications précédentes, **caractérisé en ce que** le premier élément d'accouplement (17a) est disposé sur le porte-outil (12), de préférence dans sa zone arrière.

5. Organe moteur selon l'une quelconque des revendications précédentes, **caractérisé en ce que** des surfaces d'introduction (22a, 22b) ou surfaces d'exécution (23a, 23b) obliques ou arrondies sont disposées sur les extrémités avant et/ou arrière du premier élément d'accouplement (17a) et/ou du second élément d'accouplement (17b), lesquelles provoquent un évitement automatique de l'élément d'accouplement mobile (17a) lors de l'insertion ou du retrait de l'outil de la zone de l'accouplement (17).

6. Organe moteur selon la revendication 5, **caractérisé en ce que** les surfaces d'introduction (23a, 23b) et/ou les surfaces d'exécution (23a, 23b) présentent une forme de S.

7. Organe moteur selon l'une quelconque des revendications précédentes, **caractérisé en ce que** le porte-outil (12) est formé par une pince de serrage (11) dotée de plusieurs segments de serrage (11 a) séparés par des fentes longitudinales (24) débouchant du côté de l'extrémité et répartis sur la périphérie, dans la zone d'extrémité afférente de la pince de serrage (11), sachant que la pince de serrage (11) présente des secondes fentes longitudinales (21) décalées dans le sens périphérique et débouchant sur l'autre extrémité de la pince de serrage (11), lesquelles délimitent des segments de la pince de serrage (11) disposés au milieu, sachant que les premières et les secondes fentes longitudinales (24, 21) se chevauchent dans la zone de leurs extrémités tournées l'une vers l'autre.

8. Organe moteur selon la revendication 7, **caractérisé en ce qu'**un ou plusieurs des segments forment respectivement un premier élément d'accouplement (17a).
